# EUROPEAN PATENT SPECIFICATION

(11) **EP 2 857 492 B1**
(45) Date of publication and mention of the grant of the patent: **21.08.2019**
(21) Application number: 13794337.9
(22) Date of filing: 07.05.2013
(51) Int. Cl.: C12M 1/00, A01N 1/02, B65D 33/16

(54) **PACKAGE FOR CELL-CONTAINING MATERIAL AND PACKAGE CONTAINING CELL-CONTAINING MATERIAL**
PAKET FÜR ZELLENHALTIGE MATERIALIEN UND PAKET MIT ZELLENHALTIGEN MATERIALIEN
EMBALLAGE POUR MATERIAU CONTENANT DES CELLULES ET EMBALLAGE CONTENANT UN MATERIAU CONTENANT DES CELLULES

(30) Priority: 24.05.2012 JP 2012118695
(43) Date of publication of application: 08.04.2015
(73) Proprietor: JAPAN TISSUE ENGINEERING CO., LTD., Gamagori-shi, Aichi 443-0022 (JP)
(72) Inventor: SUGIURA, Makoto, Gamagori-shi, Aichi 443-0022 (JP)
(74) Representative: Kuhnen & Wacker Patent- und Rechtsanwaltsbüro PartG mbB
(86) International application number: PCT/JP2013/062826
(87) International publication number: WO 2013/175956

(56) References cited:
- WO-A2-2004/033308
- JP-A- S61 172 560
- JP-A- 2005 087 029
- JP-A- 2008 239 168
- US-A- 3 552 638
- US-A- 3 919 053
- US-A1- 2012 109 144
- US-B1- 6 312 742

## Description

### Technical Field

The present invention relates to a package for cell-containing material and a package containing cell-containing material.

### Background Art

Packages for cell-containing material have been used to contain cell-containing material, such as tissue pieces and cell suspensions. For example, JP 2005-87029 A discloses a package for cell-containing material that includes a bag-like chamber, an opening and closing portion for delivery of a tissue piece to and from the chamber, and a tissue piece clamp portion for clamping the tissue piece in the chamber.

A closure system for flexible packaging for foodstuffs, food grade materials or pharmaceuticals and formed from plastics material with having an inner bag contained within but separate from an outer bag is furthermore disclosed by US 6,312,742 B1. After filling of the inner bag, the inner bag is closed by a first heat seal along zone in a heat-sealing operation, which can be performed through the outer bag in such a way that the outer bag is not heat sealed to the inner bag at that point. The outer bag is closed by a second seal located between the first seal and the top of the bag. A frangible zone is located between the first and second heat seals to enable the outer bag to be removed from the sealed inner bag by cutting or tearing along the frangible zone.

### Summary of Invention

### Technical Problem

In sterilization of medical devices, the sterility assurance level (SAL) is used as an indicator for the existence probability of microorganisms in the medical devices after sterilization, and a sterility assurance level of 10⁻⁶ or less ensures sterility. In medical institutions, sterile materials are treated as hygienic materials, and nonsterile materials are treated as unsanitary materials.

When the package for cell-containing material described in JP 2005-87029 A contains cell-containing material in the chamber, the package becomes a package containing cell-containing material. Since an end product cell-containing material cannot be sterilized, sterility is ensured by a manufacturing process or delivery inspection. However, the outer surface of packages containing cell-containing material is exposed to the atmosphere and as such cannot have a guarantee of sterility. In general, in the case of a package that contains a pharmaceutical agent or a medical device, the package can be subjected to sterilization, such as radiation sterilization or gas sterilization, together with the end product, and have guaranteed sterility. Unlike such a pharmaceutical agent or medical device, a package containing cell-containing material that cannot be subjected to terminal sterilization requires sterilization of the outer surface of the package alone. However, considering damage to the cell-containing material, it takes a lot of time and effort to specify the conditions even if the outer surface of the package can be independently sterilized. Furthermore, even if the conditions where the cell-containing material suffers no damage can be specified, sterilization may be insufficient. Thus, the outer surface of the package containing cell-containing material cannot have sufficiently guaranteed sterility, and the package is treated as an unsanitary material in medical institutions. However, there is a demand for a package containing cell-containing material that can be treated as a hygienic material. In sterilization in the presence of cell-containing material, a slight change in the processing conditions can affect the cell-containing material. Thus, it is complicated and difficult to specify the processing conditions.

The present invention has been accomplished in order to solve these problems. It is an object of the present invention to maintain the sterility of the outer surface of a bag for containing cell-containing material. It is another object of the present invention to make it easy to specify the conditions for sterilization of the outer surface of a bag.

### Solution to Problem

In order to achieve the principal objects, a package for cell-containing material according to the present invention has employed the following means.

A package for cell-containing material according to the present invention as set forth in each of independent claims 1 and 2 is
a package for cell-containing material that is configured to contain cell-containing material, the package comprising:
an inner bag having an opening for introducing the cell-containing material into the inner bag;
an outer bag that contains the inner bag such that the opening faces outward, wherein
the periphery of the outer bag is sealed except for an opening;
a portion of the opening outside the inner bag is sealed to form an outer bag sealed portion;
a portion of the opening overlapping the inner bag is sealed to form an inter-bag sealed portion at which an outer surface of the opening of the inner bag is bonded to an inner surface of the outer bag in contact with the outer surface of the opening of the inner bag while the opening is kept open; and
a sterile closed space surrounded by an outer surface of the inner bag and the inner surface of the outer bag.

In the package for cell-containing material, the inner bag is contained in the outer bag such that the opening faces outward. A space surrounded by the outer surface of the inner bag and the inner surface of the outer bag is a sterile closed space. Thus, the package can be sterilized before cell-containing material is contained, and can be kept sterile. Thus, the outer surface of the inner bag for containing cell-containing material can be kept sterile. Since the package can be sterilized before cell-containing material is contained, it is relatively easy to specify the sterilization conditions without considering the influence on the cell-containing material.

In the package for cell-containing material according to the present invention as set forth in independent claim 1, the the cell-containing material can be delivered through the opening into the inner bag and, after the cell-containing material is put into the inner bag through the opening, inner surfaces of the inner bag in the opening of the inner bag can be heat-sealed to form an inner bag sealed portion. The adhesive strength of heat sealing of the inner bag sealed portion is greater than the adhesive strength of the inter-bag sealed portion, allowing the outer bag to be removed from the inner bag while the closed state of the inner bag is maintained. After the cell-containing material is put into the inner bag, the inner bag can be sealed by heat-sealing the opening to provide a package containing cell-containing material. Thus, a package containing cell-containing material includes such a package for cell-containing material, the cell-containing material in the inner bag, and an inner bag sealed portion at which the opening is heat-sealed such that the interior of the inner bag forms a closed space. Even when the outer surface of the package containing cell-containing material gets dirty during transport, the outer bag can be removed in a clean room after the transport, and the bare inner bag can be treated as a hygienic material. The adhesive strength of the inter-bag sealed portion, that is, the adhesive strength between the inner bag and the outer bag is lower than the adhesive strength of the inner bag sealed portion. Thus, the outer bag can be removed from the inner bag while the closed state of the inner bag is maintained.

In the package for cell-containing material according to the present invention as set forth in independent claim 2, the opening includes a rubber stopper portion through which a syringe containing a cell suspension can be inserted and pulled out to inject the cell suspension into the inner bag, the opening includes an inner bag sealed portion that is heat-sealed together with the rubber stopper portion such that an interior of the inner bag forms a closed space, and the inner bag sealed portion is sealed with higher adhesive strength than the inter-bag sealed portion, allowing the inter-bag sealed portion and the outer bag sealed portion to be torn while the inner bag sealed portion remains sealed. When the cell-containing material is put into the inner bag, a package containing cell-containing material is provided. Thus, a package containing cell-containing material includes such a package for cell-containing material and the cell-containing material in the inner bag. Even when the outer surface of the outer bag of the package containing cell-containing material gets dirty during transport, the outer bag can be removed in a clean room after the transport, and the bare inner bag can be treated as a hygienic material. The adhesive strength of the inter-bag sealed portion, that is, the adhesive strength between the inner bag and the outer bag is lower than the adhesive strength of the inner bag sealed portion. Thus, the outer bag can be easily removed from the inner bag while the closed state of the inner bag is maintained.

### Brief Description of Drawings

Fig. 1 is a perspective view of a package 10 for cell-containing material.
Fig. 2 is a manufacturing process drawing of the package 10 for cell-containing material.
Fig. 3 is an explanatory view of packaging of a tissue piece T in the package 10 for cell-containing material.
Fig. 4 is an explanatory view of taking out an inner bag 12 from a package 34 containing cell-containing material.
Fig. 5 is a perspective view of a package 50 for cell-containing material.
Fig. 6 is a perspective view of a package 56 containing cell-containing material.
Fig. 7 is a cross-sectional view of a seal structure of the inner bag 12 and an outer bag 16.
Fig. 8 is an explanatory view of a package 134 containing cell-containing material after a tissue piece T is put into the inner bag 12 of the package 10 for cell-containing material.

### Detailed Description

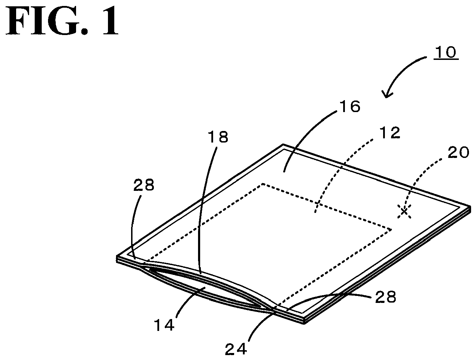

Embodiments and aspects of the present invention will be described below with reference to the accompanying drawings. Fig. 1 is a perspective view of a package 10 for cell-containing material.

The package 10 for cell-containing material is a package configured to contain cell-containing material, such as tissue pieces and cell suspensions, and includes an inner bag 12 and an outer bag 16, as illustrated in Fig. 1.

The periphery of the inner bag 12 except an opening 14 is sealed. Cell-containing material is delivered into the inner bag 12 through the opening 14.

The periphery of the outer bag 16 except an opening 24 is sealed. The outer bag 16 contains the inner bag 12 such that the opening 14 faces outward. A portion of the opening 24 outside the inner bag 12 is sealed and forms an outer bag sealed portion 28. A portion of the opening 24 that overlaps the inner bag 12 is sealed and forms an inter-bag sealed portion 18. In other words, the outer surface of the opening 14 of the inner bag 12 and the inner surface of the opening 24 of the outer bag 16 in contact with the outer surface of the opening 14 are bonded together and form the inter-bag sealed portion 18. Thus, a space surrounded by the outer surface of the inner bag 12 and the inner surface of the outer bag 16 is a closed space 20. This closed space 20 is sterilized and is kept sterile.

The material of the inner bag 12 and the material of the outer bag 16 are selected such that the adhesive strength of heat sealing of the inner surfaces of the opening 14 of the inner bag 12 is greater than the adhesive strength of heat sealing of the outer surface of the inner bag 12 and the inner surface of the outer bag 16 or the adhesive strength of heat sealing of the inner surfaces of the opening 24 of the outer bag 16. Examples of the resin material for use in the inner bag 12 or the outer bag 16 include polyvinyl chloride (PVC), polypropylene (PP), polyethylene (PE), ethylene-propylene (EP), ethylene-vinyl acetate (E/VAC), ethylene-vinyl alcohol (E/VAL), polyacrylonitrile (PAN), polystyrene (PS), polymethyl methacrylate (PMMA), polymethyl acrylate (PMA), polyamide (PA), and polyethylene terephthalate (PET). The material of the inner bag 12 is preferably different from the material of the outer bag 16. For example, the material of the inner bag 12 may be ethylene-vinyl acetate (E/VAC), and the material of the outer bag 16 may be polyethylene (PE). Alternatively, the material of the inner bag 12 may be polyvinyl chloride (PVC), and the material of the outer bag 16 may be polypropylene (PP).

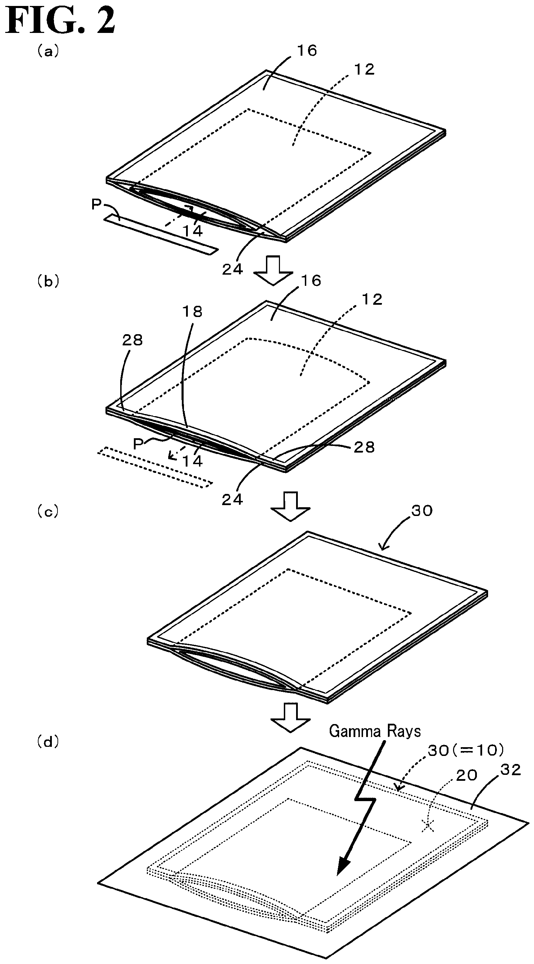

A method for manufacturing the package 10 for cell-containing material will be described below with reference to Fig. 2. First, the inner bag 12 having the opening 14 and the outer bag 16 having the opening 24 are prepared. The inner bag 12 is placed in the outer bag 16 such that the opening 14 faces outward, and the opening 14 is superimposed on the opening 24 (see Fig. 2(a)). A piece of paper P having a width of the opening 14 of the inner bag is inserted into the opening 14. The upper and lower surfaces of the opening 24 of the outer bag 16 are then placed in a heat press. In a portion where the inner bag 12 is disposed inside the opening 24 of the outer bag 16, the outer surface of the opening 14 of the inner bag 12 and the inner surface of the outer bag 16 in contact with the outer surface of the opening 14 are heat-sealed and form the inter-bag sealed portion 18. In a portion where the inner bag 12 is not disposed inside the opening 24 of the outer bag 16, the inner surfaces of the outer bag 16 are heat-sealed and form the outer bag sealed portion 28 (see Fig. 2(b)). The opening 14 of the inner bag 12 into which the piece of paper P has been inserted is not heat-sealed and is kept opened. As a result, a double bag 30 is formed (see Fig. 2(c)). The double bag 30 is placed in a large bag 32. The large bag 32 is sealed and is irradiated with gamma rays from above to sterilize the double bag 30 (see Fig. 2(d)). Thus, the interior of the large bag 32, the interior and exterior of the inner bag 12, and the interior and exterior of the outer bag 16 become sterile. In other words, the double bag 30 subjected to sterilization includes a sterile closed space 20 and serves as a package 10 for cell-containing material.

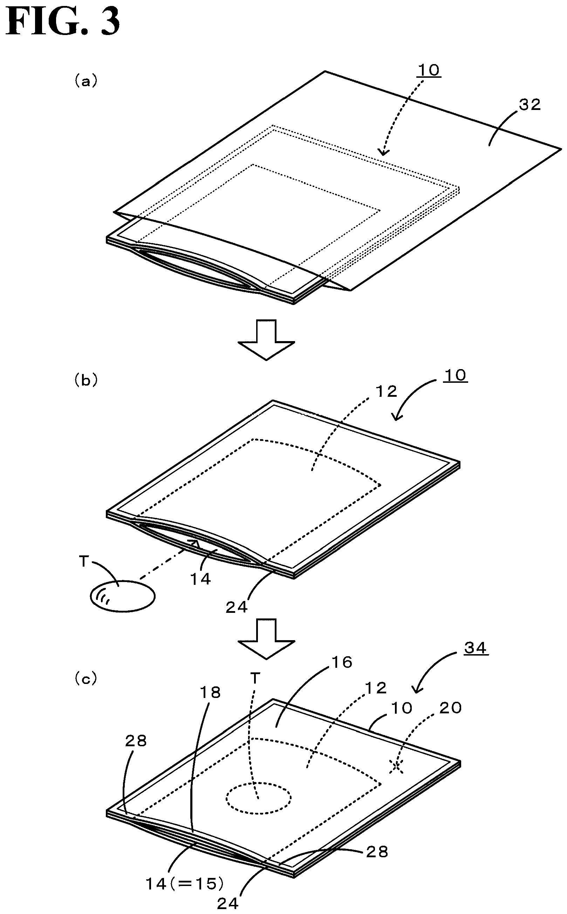

Packaging of a tissue piece T in the package 10 for cell-containing material will be described below with reference to Fig. 3. First, the large bag 32 is opened in a clean area, such as in a safety cabinet or a clean bench, and the package 10 for cell-containing material is taken out (see Fig. 3(a)). The opening 14 of the inner bag 12 of the package 10 for cell-containing material is opened. A tissue piece T after cell culture is put into the inner bag 12 through the opening 14 (see Fig. 3(b)). The upper and lower surfaces of the opening 24 of the outer bag 16 are then placed in a heat press. The inner surfaces of the inner bag 12 in the opening 14 of the inner bag 12 are heat-sealed and form an inner bag sealed portion 15, thus completing a package 34 containing cell-containing material (see Fig. 3(c)). The package 34 containing cell-containing material retains the inter-bag sealed portion 18 between the outer surface of the opening 14 of the inner bag 12 and the inner surface of the outer bag 16 in contact with the outer surface of the opening 14 and also retains the outer bag sealed portion 28 in the portion where the inner bag 12 is not disposed inside the opening 24 of the outer bag 16. The inner bag sealed portion 15 has higher adhesive strength than the inter-bag sealed portion 18 and the outer bag sealed portion 28.

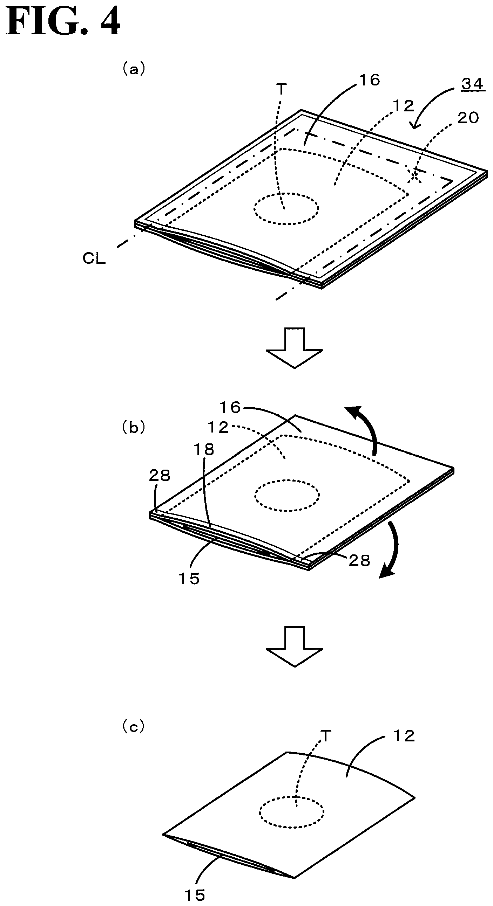

One example of an operation of taking out the inner bag 12 containing a tissue piece T from the package 34 containing cell-containing material will be described below with reference to Fig. 4. First, in a medical site, an unhygienic operator cuts the periphery of the outer bag 16 along a cutting line CL except a portion of the outer bag 16 in contact with the inner bag 12 (see Fig. 4(a)). The upper and lower surfaces of the outer bag 16 are then pulled upward and downward, respectively, to separate the outer bag 16 from the inner bag 12 (see Fig. 4(b)). The inter-bag sealed portion 18 and the outer bag sealed portion 28 have lower adhesive strength than the inner bag sealed portion 15. Thus, the inter-bag sealed portion 18 and the outer bag sealed portion 28 can be torn while the inner bag sealed portion 15 remains sealed, and the upper and lower surfaces of the outer bag 16 can be separated from the inner bag 12. As a result, the inner bag 12 is taken out (see Fig. 4(c)). Since the closed space 20 between the outer surface of the inner bag 12 and the inner surface of the outer bag 16 is sterile, the outer surface of the inner bag 12 is also kept sterile. Thus, hygienic operators can handle the inner bag 12 taken out in this manner as a hygienic material in medical sites. The inner bag 12 is opened by a hygienic operator and is transferred to a hygienic area,
such as a sterilized tray. The tissue piece T is taken out from the inner bag 12 and is used for transplantation, for example.

In the package 10 for cell-containing material, the inner bag 12 is placed in the outer bag 16 such that the opening 14 faces outward. The closed space 20 surrounded by the outer surface of the inner bag 12 and the inner surface of the outer bag 16 is sterile, and the outer surface of the inner bag 12 for containing the tissue piece T can be kept sterile. Since the package 10 can be sterilized before the tissue piece T is put into the package 10, it is relatively easy to specify the sterilization conditions without considering the influence on the tissue piece T.

The tissue piece T can be put into and taken out from the inner bag 12 through the opening 14.

After the tissue piece T is put into the inner bag 12 of the package 10 for cell-containing material, the inner bag 12 can be sealed by heat-sealing the opening 14 to provide the package 34 containing cell-containing material. Even when the outer surface of the package 34 containing cell-containing material gets dirty during transport, the outer bag 16 can be removed in a medical site after the transport, and the bare inner bag 12 can be treated as a hygienic material. The adhesive strength of the inter-bag sealed portion 18, that is, the adhesive strength between the inner bag 12 and the outer bag 16 is lower than the adhesive strength of the inner bag sealed portion 15. Thus, the outer bag 16 can be removed from the inner bag 12 while the closed state of the inner bag 12 is maintained.

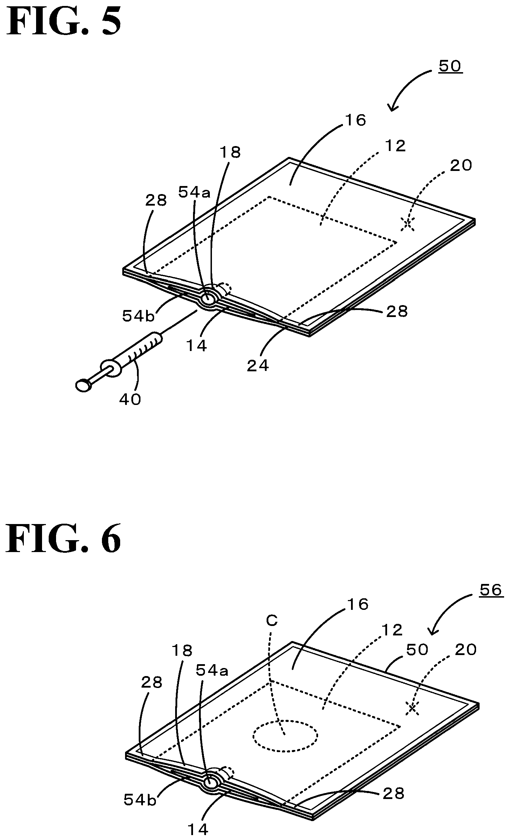

In the embodiment described above, the tissue piece T is put into the inner bag 12 through the opening 14 of the inner bag 12. For examples, in the case of packaging of a liquid substance, such as a cell suspension, instead of the tissue piece T is put into the inner bag, as in a package 50 for cell-containing material illustrated in Fig. 5, a rubber stopper portion 54a and an inner bag sealed portion 54b may be disposed in the opening 14. A syringe 40 can be inserted and pulled out through the rubber stopper portion 54a. The opening 14 is heat-sealed together with the rubber stopper portion 54a such that the interior of the inner bag 12 becomes a closed space, and forms the inner bag sealed portion 54b. This also allows the outer surface of the inner bag 12 to be kept sterile. In this case, the inner bag sealed portion 54b is sealed with higher adhesive strength than the inter-bag sealed portion 18 and the outer bag sealed portion 28. The same components of the package 50 for cell-containing material as in the package 10 for cell-containing material are denoted by the same reference numerals and will not be described again. In the same manner as in the embodiment described above, the package 50 for cell-containing material is placed in a large bag and is irradiated with gamma rays in order to sterilize the closed space 20 surrounded by the outer surface of the inner bag 12 and the inner surface of the outer bag 16 and sterilize the interior of the inner bag 12. When a cell suspension is packaged in the package 10 for cell-containing material, the cell suspension may be packaged in the same manner as in the embodiment described above. Although the opening 14 is heat-sealed after the tissue piece T is put into the inner bag 12 through the opening 14 in the embodiment, the needle attached to the syringe 40 containing a cell suspension is inserted through the rubber stopper portion 54a, and the cell suspension C is injected into the inner bag 12 with the syringe 40. When the inner bag 12 is taken out from the package 50 for cell-containing material that contains the cell suspension C (that is, a package 56 containing cell-containing material, see Fig. 6), in the same manner as in the embodiment, the inner bag 12 may be taken out from the package 50 for cell-containing material by cutting and removing the outer bag 16. Also in this case, the inter-bag sealed portion 18 and the outer bag sealed portion 28 have lower adhesive strength than the inner bag sealed portion 54b. Thus, the inter-bag sealed portion 18 and the outer bag sealed portion 28 can be torn while the inner bag sealed portion 54b remains sealed, and the inner bag 12 can be taken out from the package 50 for cell-containing material. Since the closed space 20 between the outer surface of the inner bag 12 and the inner surface of the outer bag 16 is sterile, the outer surface of the inner bag 12 is also kept sterile. Thus, the inner bag 12 taken out in this manner can be treated as a hygienic material in medical institutions.

Although the tissue piece T after cell culture is used in the embodiment described above, the tissue piece T before cell culture may be used. In this case, the tissue piece T may be cultured in the inner bag 12 before sealing the opening 14. The package 56 containing cell-containing material illustrated in Fig. 6 may be transported to an intended institution after the cell suspension C is cultured in the inner bag 12, and the inner bag 12 may be taken out in a clean area in the institution. In the embodiment illustrated in Fig. 5, a scaffold, such as a porous body, may be enclosed and sterilized in the inner bag 12 in advance, and the cell suspension injected in the inner bag 12 may be cultured on the scaffold under sterile conditions.

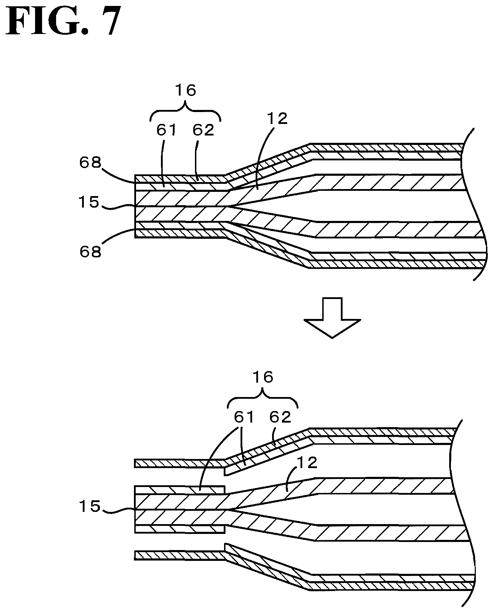

In the embodiment described above, the material of the inner bag 12 and the material of the outer bag 16 are selected such that the adhesive strength of the inner bag sealed portion 15 is greater than the adhesive strength of the inter-bag sealed portion 18 and the outer bag sealed portion 28. As illustrated in Fig. 7, the outer bag 16 may be composed of a plurality of layers (two layers in this case) formed of different materials. An inner layer 61 of the outer bag 16 may be formed of a material that can be strongly welded to the inner bag 12 (a material that can be welded with adhesive strength comparable to the adhesive strength of the inner bag sealed portion 15). An outer layer 62 of the outer bag 16 may be bonded to the inner layer 61 with adhesive strength lower than the adhesive strength of the inner bag sealed portion 15. In such a structure, when the outer bag 16 is cut and removed from the inner bag 12, the inner bag sealed portion 15 is kept sealed, the inner layer 61 of the outer bag 16 remains on the inner bag 12, and the inner layer 61 is separated from the outer layer 62. In such a seal structure, a bonded portion 68 between the inner layer 61 and the outer layer 62 of the outer bag 16 corresponds to an inter-bag sealed portion of the present invention. In the case where the outermost layer of the outer bag 16 can be torn off, even if a portion of the outer bag 16 other than the outermost layer remains on the inner bag 12, the outer surface of the inner bag 12 taken out from the outer bag 16 remains sterile.

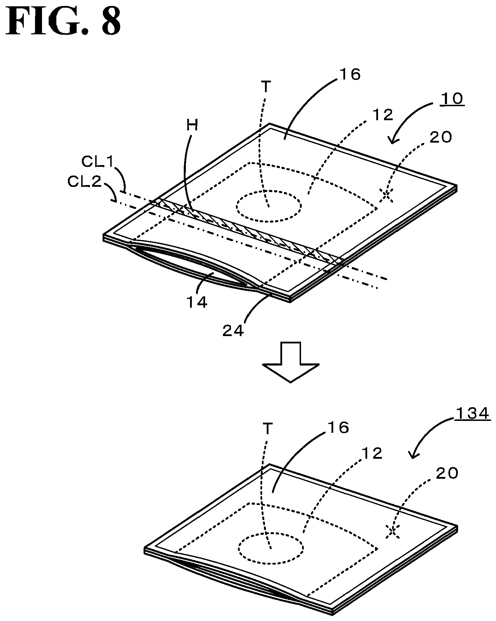

In the embodiment described above, the tissue piece T is put into the package 10 for cell-containing material, and both the top face and the bottom face of the opening 24 of the outer bag 16 are then heat-pressed. However, another portion may be heat-pressed. For example, as illustrated in Fig. 8, a heat press region H between the opening 24 of the outer bag 16 and the tissue piece T (see the hatched portion in Fig. 8) may be heat-pressed, and then the package 10 may be cut along a cutting line CL1 on the heat press region H, thereby providing a package 134 containing cell-containing material. Also in this case, the closed space 20 remains sterile, and the tissue piece T is contained in the inner bag 12. Among heat-pressed portions, opposite portions of the inner bag 12 or opposite portions of the outer bag 16 can be strongly welded because the same material is heat-sealed. On the other hand, a contact portion between the outer surface of the inner bag 12 and the inner surface of the outer bag 16 is weakly welded because different materials are heat-sealed. Thus, also in the package 134 containing cell-containing material, the outer bag 16 can be removed from the inner bag 12 while the closed state of the inner bag 12 is maintained. Instead of cutting along the cutting line CL1, the package 10 may be cut along a cutting line CL2 between the opening 24 and the heat press region H.

Although each of the sealed portions is formed by heat sealing in the embodiment described above, the sealed portions may be formed using another bonding method other than heat sealing. For example, an adhesive may be used.

### Industrial Applicability

The present invention is applicable to a field of, for example, regenerative medicine.

### Reference Signs List

Package 10 for cell containing material, inner bag 12, opening 14, inner bag sealed portion 15, outer bag 16, inter-bag sealed portion 18, closed space 20, opening 24, outer bag sealed portion 28, double bag 30, large bag 32, package 34 containing cell-containing material, syringe 40, package 50 for cell-containing material, rubber stopper portion 54a, inner bag sealed portion 54b, package 56 containing cell-containing material, layers 61, 62, bonded portion 68, package 134 containing cell-containing material, cell suspension C, cutting lines CL, CL1, CL2, heat press region H, paper P, and tissue piece T.

## Claims

1. A package (10; 50) for cell-containing material, comprising:
an inner bag (12) having an opening (14) for introducing the cell-containing material into the inner bag (12);
an outer bag (16) that contains the inner bag (12) such that the opening (14) faces outward, wherein
the periphery of the outer bag (16) is sealed except for an opening (24);
a portion of the opening (24) outside the inner bag (12) is sealed to form an outer bag sealed portion (28);
a portion of the opening (24) overlapping the inner bag (12) is sealed to form an inter-bag sealed portion (18) at which an outer surface of the opening (14) of the inner bag (12) is bonded to an inner surface of the outer bag (16) in contact with the outer surface of the opening of the inner bag (12) while the opening (14) is kept open;
a sterile closed space (20) surrounded by an outer surface of the inner bag (12) and the inner surface of the outer bag (16),
wherein
the cell-containing material can be delivered through the opening (14) into the inner bag (12) and, after the cell-containing material is put into the inner bag (12) through the opening (14), inner surfaces of the inner bag (12) in the opening (14) of the inner bag (12) can be heat-sealed to form an inner bag sealed portion (15),
the adhesive strength of the inner bag sealed portion (15) is greater than the adhesive strength of the inter-bag sealed portion (18), allowing the outer bag (16) to be removed from the inner bag (12) while the closed state of the inner bag (12) is maintained.

2. A package (10; 50) for cell-containing material, comprising:
an inner bag (12) having an opening (14) for introducing the cell-containing material into the inner bag (12);
an outer bag (16) that contains the inner bag (12) such that the opening (14) faces outward, wherein
the periphery of the outer bag (16) is sealed except for an opening (24);
a portion of the opening (24) outside the inner bag (12) is sealed to form an outer bag sealed portion (28); and
a portion of the opening (24) overlapping the inner bag (12) is sealed to form an inter-bag sealed portion (18) at which an outer surface of the opening (14) of the inner bag (12) is bonded to an inner surface of the outer bag (16) in contact with the outer surface of the opening (14) of the inner bag (12); and
a sterile closed space (20) surrounded by an outer surface of the inner bag (12) and the inner surface of the outer bag (16),
wherein
the opening (14) includes a rubber stopper portion (54a) through which a syringe_containing a cell suspension can be inserted and pulled out to inject the cell suspension into the inner bag (12),
the opening (14) includes an inner bag sealed portion (54b) that is heat-sealed together with the rubber stopper portion (54a) such that an interior of the inner bag (12) forms a closed space, and
the inner bag sealed portion (54b) is sealed with higher adhesive strength than the inter-bag sealed portion (18), allowing the inter-bag sealed portion (18) and the outer bag sealed portion (28) to be torn while the inner bag sealed portion (54b) remains sealed.

3. A package (10; 50) containing cell-containing material, comprising:
a package (10; 50) for cell-containing material according to Claim 1;
cell-containing material in the inner bag (12); and
an inner bag sealed portion (15) at which the opening is heat-sealed such that an interior of the inner bag (12) forms a closed space.

4. A package (10; 50) containing cell-containing material, comprising:
the package (10; 50) for cell-containing material according to Claim 2; and
cell-containing material in the inner bag (12).

## Patentansprüche

1. Verpackung (10; 50) für zellhaltiges Material, aufweisend:
einen inneren Beutel (12) mit einer Öffnung (14) zum Einführen des zellhaltigen Materials in den inneren Beutel (12);
einen äußeren Beutel (16), welcher den inneren Beutel (12) enthält, sodass die Öffnung (14) nach außen zeigt, wobei
die Außenfläche des äußeren Beutels (16) bis auf eine Öffnung (24) versiegelt ist;
ein Abschnitt der Öffnung (24) außerhalb des inneren Beutels (12) versiegelt ist, um einen versiegelten Abschnitt (28) des äußeren Beutels zu bilden;
ein Abschnitt der Öffnung (24), der den inneren Beutel (12) überlappt, versiegelt ist, um einen versiegelten Zwischenbeutelabschnitt (18) zu bilden, an dem eine äußere Fläche der Öffnung (14) des inneren Beutels (12) mit einer inneren Fläche des äußeren Beutels (16), welche die äußere Fläche der Öffnung des inneren Beutels (12) berührt, verbunden ist, während die Öffnung (14) offen gehalten wird;
einen sterilen geschlossener Raum (20), umgeben von einer äußeren Fläche des inneren Beutels (12) und der inneren Fläche des äußeren Beutels (16),
wobei
das zellhaltige Material durch die Öffnung (14) in den inneren Beutel (12) geliefert werden kann und, nachdem das zellhaltige Material durch die Öffnung (14) in den inneren Beutel (12) abgelegt worden ist, innere Flächen des inneren Beutels (12) in der Öffnung (14) des inneren Beutels (12) heißversiegelt werden können, um einen versiegelten Abschnitt (15) des inneren Beutels zu bilden, wobei
die Haftfestigkeit des versiegelten Abschnitts (15) des inneren Beutels größer ist als die Haftfestigkeit des versiegelten Zwischenbeutelabschnitts (18), was das Entfernen des äußeren Beutels (16) vom inneren Beutel (12) erlaubt, während der geschlossene Zustand des inneren Beutels (12) erhalten bleibt.

2. Verpackung (10; 50) für zellhaltiges Material, aufweisend:
einen inneren Beutel (12) mit einer Öffnung (14) zum Einführen des zellhaltigen Materials in den inneren Beutel (12);
einen äußeren Beutel (16), welcher den inneren Beutel (12) enthält, sodass die Öffnung (14) nach außen zeigt, wobei
die Außenfläche des äußeren Beutels (16) bis auf eine Öffnung (24) versiegelt ist;
ein Abschnitt der Öffnung (24) außerhalb des inneren Beutels (12) versiegelt ist, um einen versiegelten Abschnitt (28) des äußeren Beutels zu bilden; und
ein Abschnitt der Öffnung (24), der den inneren Beutel (12) überlappt, versiegelt ist, um einen versiegelten Zwischenbeutelabschnitt (18) zu bilden, an dem eine äußere Fläche der Öffnung (14) des inneren Beutels (12) mit einer inneren Fläche des äußeren Beutels (16), welche die äußere Fläche der Öffnung des inneren Beutels (12) berührt, verbunden ist; und
einen sterilen geschlossener Raum (20), umgeben von einer äußeren Fläche des inneren Beutels (12) und der inneren Fläche des äußeren Beutels (16),
wobei
die Öffnung (14) einen Gummistopfen-Abschnitt (54a) beinhaltet, durch den eine Spritze, die eine Zellsuspension enthält, eingeschoben und herausgezogen werden kann, um die Zellsuspension in den inneren Beutel (12) einzuspritzen,
die Öffnung (14) einen versiegelten Abschnitt (54b) des inneren Beutels beinhaltet, der zusammen mit dem Gummistopfen-Abschnitt (54a) heißversiegelt ist, sodass ein Innenraum des inneren Beutels (12) einen geschlossenen Raum bildet, und
der versiegelte Abschnitt (54b) des inneren Beutels mit höherer Haftfestigkeit versiegelt ist als der versiegelte Zwischenbeutelabschnitt (18), was dem versiegelten Zwischenbeutelabschnitt (18) und dem versiegelten Abschnitt (28) des äußeren Beutels erlaubt zu reißen, während der versiegelte Abschnitt (54b) des inneren Beutels versiegelt bleibt.

3. Verpackung (10; 50), die zellhaltiges Material enthält, aufweisend:
eine Verpackung (10; 50) für zellhaltiges Material nach Anspruch 1;
zellhaltiges Material im inneren Beutel (12); und
einen versiegelten Abschnitt (15) des inneren Beutels, an dem die Öffnung heißversiegelt ist, sodass ein Innenraum des inneren Beutels (12) einen geschlossenen Raum bildet.

4. Verpackung (10; 50), die zellhaltiges Material enthält, aufweisend:
die Verpackung (10; 50) für zellhaltiges Material nach Anspruch 2; und
zellhaltiges Material im inneren Beutel (12).

## Revendications

1. Emballage (10 ; 50) pour une substance contenant des cellules, comprenant :
un sac intérieur (12) ayant une ouverture (14) pour introduire la substance contenant des cellules dans le sac intérieur (12) ;
un sac extérieur (16) qui contient le sac intérieur (12) de sorte que l'ouverture (14) soit face à l'extérieur, dans lequel
la périphérie du sac extérieur (16) est scellée à l'exception d'une ouverture (24) ;
une partie de l'ouverture (24) à l'extérieur du sac intérieur (12) est scellée pour former une partie scellée de sac extérieur (28) ;
une partie de l'ouverture (24) chevauchant le sac intérieur (12) est scellée pour former une partie scellée inter-sacs (18) au niveau de laquelle une surface extérieure de l'ouverture (14) du sac intérieur (12) est attachée à une surface intérieure du sac extérieur (16) en contact avec la surface extérieure de l'ouverture du sac intérieur (12) alors que l'ouverture (14) est maintenue ouverte ;
un espace fermé stérile (20) entouré par une surface extérieure du sac intérieur (12) et la surface intérieure du sac extérieur (16),
dans lequel
la substance contenant des cellules peut être fournie à travers l'ouverture (14) dans le sac intérieur (12) et, après que la substance contenant une cellule est mise dans le sac intérieur (12) à travers l'ouverture (14), des surfaces intérieures du sac intérieur (12) dans l'ouverture (14) du sac intérieur (12) peuvent être thermoscellées pour former une partie scellée de sac intérieur (15),
la force d'adhérence de la partie scellée de sac intérieur (15) est supérieure à la force d'adhérence de la partie scellée inter-sacs (18), permettant au sac extérieur (16) d'être retiré du sac intérieur (12) alors que l'état fermé du sac intérieur (12) est maintenu.

2. Emballage (10 ; 50) pour une substance contenant des cellule, comprenant :
un sac intérieur (12) ayant une ouverture (14) pour introduire la substance contenant des cellules dans le sac intérieur (12) ;
un sac extérieur (16) qui contient le sac intérieur (12) de sorte que l'ouverture (14) soit face à l'extérieur, dans lequel
la périphérie du sac extérieur (16) est scellée à l'exception d'une ouverture (24) ;
une partie de l'ouverture (24) à l'extérieur du sac intérieur (12) est scellée pour former une partie scellée de sac extérieur (28) ; et
une partie de l'ouverture (24) chevauchant le sac intérieur (12) est scellée pour former une partie scellée inter-sacs (18) au niveau de laquelle une surface extérieure de l'ouverture (14) du sac intérieur (12) est attachée à une surface intérieure du sac extérieur (16) en contact avec la surface extérieure de l'ouverture (14) du sac intérieur (12) ; et
un espace fermé stérile (20) entouré par une surface extérieure du sac intérieur (12) et la surface intérieure du sac extérieur (16),
dans lequel
l'ouverture (14) inclut une partie de bouchon en caoutchouc (54a) à travers laquelle une seringue contenant une suspension cellulaire peut être insérée et retirée pour injecter la suspension cellulaire dans le sac intérieur (12),
l'ouverture (14) inclut une partie scellée de sac intérieur (54b) qui est thermoscellée avec la partie de bouchon en caoutchouc (54a) de sorte qu'un intérieur du sac intérieur (12) forme un espace fermé, et
la partie scellée de sac intérieur (54b) est scellée avec une force d'adhérence supérieure à la partie scellée inter-sacs (18), permettant à la partie scellée inter-sacs (18) et à la partie scellée de sac extérieur (28) d'être déchirées alors que la partie scellée de sac intérieur (54b) reste scellée.

3. Emballage (10 ; 50) contenant une substance contenant des cellules, comprenant :
un emballage (10 ; 50) pour une substance contenant des cellules selon la revendication 1 ;
une substance contenant des cellules dans le sac intérieur (12) ; et
une partie scellée de sac intérieur (15) à laquelle l'ouverture est thermoscellée de sorte qu'un intérieur du sac intérieur (12) forme un espace fermé.

4. Emballage (10; 50) contenant une substance contenant des cellules, comprenant :
l'emballage (10; 50) pour une substance contenant des cellules selon la revendication 2 ; et
une substance contenant des cellules dans le sac intérieur (12).
